# FASCICULE DE BREVET EUROPEEN

(11) **EP 0 975 567 B1**
(45) Date de publication et mention de la délivrance du brevet: **10.04.2002**
(21) Numéro de dépôt: 98902051.6
(22) Date de dépôt: 09.01.1998
(51) Int. Cl.: C07C 17/358, C07C 19/08

(54) **ISOMERISATION D'HYDROFLUOROCARBURES**
ISOMERISIERUNG VON FLUORKOHLENWASSERSTOFFEN
ISOMERIZATION OF HYDROFLUOROCARBON

(30) Priorité: 17.01.1997 FR 9700477
(43) Date de publication de la demande: 02.02.2000
(73) Titulaire: Atofina, 92800 Puteaux (FR)
(72) Inventeur: HUB, Serge, F-69100 Villeurbanne (FR); GUILLET, Dominique, F-69390 Vernaison (FR)
(86) Numéro de dépôt international: FR9800035
(87) Numéro de publication internationale: WO9831650

(56) Documents cités:
- EP-A- 0 365 296
- US-A- 2 315 871
- US-A- 4 902 838

## Description

La présente invention qui concerne le domaine des hydrocarbures fluorés a pour objet l'isomérisation d'hydrofluorocarbures (HFC) et, plus particulièrement, celle du 1,1,2,2-tétrafluoroéthane (F134) en 1,1,1,2-tétrafluoroéthane (F134a).

Le F134a est l'un des hydrofluorocarbures (HFC) retenus dans le cadre de la substitution des chlorofluorocarbures (CFC) et des hydrochlorofluorocarbures (HCFC) qui sont déjà interdits ou en cours d'interdiction en raison de leur effet nocif sur la couche d'ozone stratosphérique.

Plusieurs voies d'accès au F134a sont connues, à savoir :
- la fluoration en phase gazeuse ou liquide du 1-chloro-2,2,2-trifluoroéthane (F133a),
- la fluoration en phase liquide du trichloroéthylène,
- l'hydrogénolyse du 1,1-dichloro-1,2,2,2-tétrafluoroéthane (F114a) ou du 1-chloro-1,2,2,2-tétrafluoroéthane (F124),
- l'isomérisation du 1,1,2,2-tétrafluoroéthane (F134).

Selon la littérature, ce dernier procédé s'effectue en utilisant des catalyseurs. Ainsi, les brevets EP 365 296 et JP 03 261731 décrivent l'utilisation de catalyseurs à base de chrome et le brevet US 4 902 838 revendique un catalyseur du type alumine fluorée ; le brevet JP 02 115135 préfère utiliser un catalyseur du type chlorofluorure d'aluminium. L'utilisation d'un catalyseur ne suffit pas toujours ; ainsi, le brevet US 4 902 838 préconise l'introduction d'oxygène dans le milieu afin de maintenir l'activité catalytique dans le temps, et la demande de brevet WO 95/15300 préconise l'introduction d'une source d'ions chlorure.

Il a maintenant été trouvé que l'on peut isomériser le F134 en F134a sans l'aide d'un catalyseur par un simple traitement thermique en présence d'hydrogène. Cette méthode peut s'appliquer aussi à l'isomérisation d'autres HFC, par exemple à celle du 1,1,2-trifluoroéthane (F143) en 1,1,1-trifluoroéthane (F143a) ou à celle du 1,2-difluoroéthane (F152) en 1,1-difluoroéthane (F152a).

La présente invention a donc pour objet un procédé d'isomérisation d'un hydrofluorocarbure présentant une certaine stabilité thermodynamique (HFC 1) en un hydrofluorocarbure de plus grande stabilité thermodynamique (HFC 2), caractérisé en ce que l'on soumet l'hydrofluorocarbure HFC 1 à un traitement thermique en présence d'hydrogène à une température supérieure à 500°C.

Le procédé selon l'invention est avantageusement réalisé à une température comprise entre 500 et 1000°C, de préférence entre 600 et 750°C.

La pression de la réaction peut être comprise entre 0,1 et 50 bars, mais on préfère travailler entre la pression atmosphérique et 20 bars.

Le rapport molaire H₂/HFC 1 peut aller de 1 à 100, mais on préfère généralement travailler à un rapport molaire compris entre 2 et 20. Le flux des réactifs (HFC 1 et H₂) entrant dans le réacteur peut être dilué par un gaz inerte tel que l'hélium ou l'azote.

Le temps de séjour des réactifs dans la partie chaude du réacteur peut varier dans de larges limites. Il varie en sens inverse de la température et est généralement compris entre 0,1 et 1000 secondes, de préférence entre 1 et 300 secondes.

L'isomérisation peut être réalisée dans un réacteur vide, c'est-à-dire un réacteur qui ne contient aucun garnissage mais peut cependant être équipé de thermocouples et de chicanes. Le réacteur peut être en quartz ou métallique. Dans ce cas, le métal du matériau constituant le réacteur peut être choisi parmi les métaux tels que le nickel, le fer, le titane, le chrome, le molybdène, le cobalt, l'or ou leurs alliages. Le métal, choisi plus particulièrement pour limiter la corrosion ou les phénomènes catalytiques, peut être massif ou plaqué sur un autre métal, comme dans le cas d'un réacteur doré sur sa surface interne.

Les exemples suivants illustrent l'invention sans la limiter.

### EXEMPLES 1 à 3

Les essais ont été réalisés à pression atmosphérique dans un réacteur tubulaire en quartz de 47 cm de longueur et de 2,1 cm de diamètre interne, placé dans un four électrique d'une puissance de 1,5 kW; la température du four était mesurée à l'aide d'un thermocouple.

Les réactifs (F134 et H₂) étaient introduits simultanément par l'intermédiaire de débitmètres massiques permettant le contrôle des débits et donc des rapports molaires.

L'analyse des produits gazeux a été faite par chromatographie (CPV) en ligne sur la sortie du réacteur.

Le tableau suivant résume les conditions opératoires et les résultats obtenus.

| **EXEMPLE** | **1** | **2** | **3** |
|---|---|---|---|
| **Conditions opératoires :** | | | |
| - température (°C) | 700 | 700 | 700 |
| - débit H₂ (mmole/h) | 509 | 187,1 | 29,9 |
| - débit F134 (mmole/h) | 50,4 | 96 | 31,7 |
| - temps de séjour (s) | 10 | 21 | 95 |

| **Résultats** | | | |
|---|---|---|---|
| - conversion du F134 | 23 % | 27 % | 47 % |
| - sélectivité en F134a | 81 % | 82 % | 81 % |

### EXEMPLES 4 à 6

Les essais ont été réalisés à pression atmosphérique dans un réacteur tubulaire en quartz de 47 cm de longueur et de 1,5 cm de diamètre interne, placé dans un four électrique d'une puissance de 1,5 kW ; la température du four était mesurée à l'aide d'un thermocouple.

Le tableau suivant résume les conditions opératoires et les résultats obtenus :

| **EXEMPLE** | **4** | **5** | **6** |
|---|---|---|---|
| **Conditions opératoires :** | | | |
| - température (°C) | 700 | 700 | 700 |
| - débit H₂ (mmole/h) | 301,3 | 141,5 | 59,8 |
| - débit F134 (mmole/h) | 15,2 | 15,2 | 15,2 |
| - temps de séjour (s) | 4,9 | 9,9 | 20,6 |

| **Résultats** | | | |
|---|---|---|---|
| - conversion du F134 | 13 % | 24 % | 38 % |
| - sélectivité en F134a | 84 % | 78 % | 79 % |

### EXEMPLE 7

On a opéré comme dans les exemples 1 à 3, mais en remplaçant le réacteur de quartz par un réacteur en Inconel 600 de mêmes dimensions. En travaillant dans les conditions opératoires suivantes:
- température 700°C
- débit H₂ 29,9 mmole/h
- débit F134 31,7 mmole/h
- temps de séjour 95 secondes
on a obtenu une conversion du F134 de 44 % et une sélectivité en F134a égale à 74 %.

### EXEMPLES 8 et 9 comparatifs

On a opéré de la même manière que dans les exemples 1 à 3, mais en remplaçant l'hydrogène par l'hélium. Le tableau suivant résume les conditions opératoires et les résultats obtenus.

| **EXEMPLE** | **8** | **9** |
|---|---|---|
| **Conditions opératoires :** | | |
| - température (°C) | 700 | 700 |
| - débit He (mmole/h) | 312,1 | 35,3 |
| - débit F134 (mmole/h) | 22,3 | 31,7 |
| - temps de séjour (s) | 18 | 95 |

| **Résultats** | | |
|---|---|---|
| - conversion du F134 | 3 % | 15 % |
| - sélectivité en F134a | 33 % | 60 % |

## Revendications

1. Procédé d'isomérisation d'un hydrofluorocarbure présentant une certaine stabilité thermodynamique (HFC 1) en un hydrofluorocarbure de plus grande stabilité thermodynamique (HFC 2), **caractérisé en ce que** l'on soumet l'hydrofluorocarbure (HFC 1) à un traitement thermique en présence d'hydrogène à une température supérieure à 500°C.

2. Procédé selon la revendication 1 dans lequel on opère à une température comprise entre 500 et 1000°C, de préférence entre 600 et 750°C.

3. Procédé selon la revendication 1 ou 2, dans lequel on opère à une pression comprise entre 0,1 et 50 bars, de préférence à la pression atmosphérique ou à une pression allant jusqu'à 20 bars.

4. Procédé selon l'une des revendications 1 à 3 dans lequel le rapport molaire de l'hydrogène à l'hydrofluorocarbure (HFC 1) est compris entre 1 et 100, de préférence entre 2 et 20.

5. Procédé selon l'une des revendications 1 à 4 dans lequel on travaille dans un réacteur vide, métallique ou en quartz.

6. Procédé selon l'une des revendications 1 à 5 dans lequel l'hydrofluorocarbure à isomériser (HFC 1) est le 1,1,2,2-tétrafluoroéthane.

## Claims

1. Process for the isomerization of a hydrofluorocarbon having a certain thermodynamic stability (HFC 1) into a hydrofluorocarbon of greater thermodynamic stability (HFC 2), **characterized in that** the hydrofluorocarbon (HFC 1) is subjected to a heat treatment in the presence of hydrogen at a temperature of above 500°C.

2. Process according to Claim 1, which is carried out at a temperature of between 500 and 1000°C, preferably between 600 and 750°C.

3. Process according to Claim 1 or 2, which is carried out at a pressure of between 0.1 and 50 bar, preferably at atmospheric pressure or at a pressure ranging up to 20 bar.

4. Process according to one of Claims 1 to 3, in which the molar ratio of the hydrogen to the hydrofluorocarbon (HFC 1) is between 1 and 100, preferably between 2 and 20.

5. Process according to one of Claims 1 to 4, which is carried out in an empty, metal or quartz reactor.

6. Process according to one of Claims 1 to 5, in which the hydrofluorocarbon to be isomerized (HFC 1) is 1,1,2,2-tetrafluoroethane.

## Patentansprüche

1. Verfahren zur Isomerisierung eines Fluorkohlenwasserstoffs mit einer bestimmten thermodynamischen Stabilität (FCKW 1) zu einem Fluorkohlenwasserstoff mit größerer thermodynamischer Stabilität (FCKW 2),
**dadurch gekennzeichnet,**
**daß** man den Fluorkohlenwasserstoff (FCKW 1) einer thermischen Behandlung in Gegenwart von Wasserstoff bei einer Temperatur oberhalb von 500 °C unterwirft.

2. Verfahren nach Anspruch 1, bei dem man bei einer Temperatur zwischen 500 und 1000 °C, vorzugsweise zwischen 600 und 750 °C, verfährt.

3. Verfahren nach Anspruch 1 oder 2, bei dem man bei einem Druck zwischen 0,1 und 50 bar, vorzugsweise bei Atmosphärendruck oder einem Druck von bis zu 20 bar, verfährt.

4. Verfahren nach einem der Ansprüche 1 bis 3, bei dem das Molverhältnis von Wasserstoff zu Fluorkohlenwasserstoff (FCKW 1) zwischen 1 und 100, vorzugsweise zwischen 2 und 20, liegt.

5. Verfahren nach einem der Ansprüche 1 bis 4, bei dem man in einem nicht ausgekleideten Reaktor aus Metall oder Quartz arbeitet.

6. Verfahren nach einem der Ansprüche 1 bis 5, bei dem der zu isomerisierende Fluorkohlenwasserstoff (FCKW 1) 1,1,2,2-Tetrafluorethan ist.
